# EUROPEAN PATENT SPECIFICATION

(11) **EP 4 104 790 B1**
(45) Date of publication and mention of the grant of the patent: **01.05.2024**
(21) Application number: 20927569.2
(22) Date of filing: 27.03.2020
(51) Int. Cl.: A61B 34/30, A61B 17/28, A61B 17/29

(54) **SURGICAL TOOL**
CHIRURGISCHES INSTRUMENT
INSTRUMENT CHIRURGICAL

(43) Date of publication of application: 21.12.2022
(73) Proprietor: RIVERFIELD Inc., 1070052 Tokyo (JP)
(72) Inventor: SHINDO, Koki, Tokyo 107-0052 (JP)
(74) Representative: adares Patent- und Rechtsanwälte Reininger & Partner GmbB
(86) International application number: PCT/JP2020/014157
(87) International publication number: WO 2021/192255

(56) References cited:
- WO-A1-2020/035893
- JP-A- 2009 106 606
- JP-A- 2011 139 841
- JP-A- 2011 517 421
- US-A1- 2009 024 142
- US-A1- 2011 015 650
- US-A1- 2014 001 234
- US-A1- 2019 125 464
- US-A1- 2020 060 516

## Description

### TECHNICAL FIELD

The present disclosure relates to a surgical tool mounted to a surgical robot.

### BACKGROUND ART

For example, Patent Document 1 discloses a medical support arm device that is capable of continuing a surgical operation even when power supply to the medical support arm device is stopped during the surgical operation. Patent Documents 2 to 5 disclose medical robots that can be operated manually through a structure or a mechanism that is fixedly mounted on the medical robots.

### PRIOR ART DOCUMENTS

### PATENT DOCUMENTS

Patent Document 1: Japanese Unexamined Patent Application Publication No. 2016-152906
Patent Document 2: US 2020/060516 A1
Patent Document 3: US 2019/125464 A1
Patent Document 4: US 2014/001234 A1
Patent Document 5: US 2009/024142 A1

### SUMMARY OF THE INVENTION

### PROBLEMS TO BE SOLVED BY THE INVENTION

Among those surgical tools mounted to a surgical robot, for example, a treatment instrument, such as forceps, is provided with a treatment portion such as a hand part (a gripper) on a distal end side. The treatment portion is provided with at least one movable part.

The movable part can be directly or indirectly displaced by an actuator using air pressure or electric power. Because of this, if a supply of air pressure, or, power supply is stopped, and then the actuator is stopped, a state of the movable part is retained as it is when the actuator is stopped.

Specifically, for example, when the power supply is stopped in "a state in which the gripper of forceps (a surgical tool) grips a tissue of a patient", it is difficult to continue the surgical operation or to discontinue the surgical operation.

The present disclosure discloses an example of a surgical tool that is mounted to the surgical robot, the surgical tool being capable of dealing with a case where power supply is stopped.

### MEANS FOR SOLVING THE PROBLEMS

The problems are solved by a surgical tool according to claim 1. The surgical tool mounted to the surgical robot comprises, for example, at least one of the following components.

Specifically, the components are: a treatment portion used in surgical treatment, the treatment portion including at least one movable part that can be displaced; a slider that generates a driving force to displace the movable part, the slider generating the driving force through reciprocating motion along a linear direction; and a manual releasing tool for displacing the movable part, the manual releasing tool being detachably mountable to a main body at which the slider is accommodated.

Thus, in the surgical tool, when a user mounts the manual releasing tool to the main body, the user can displace the movable part by manual operation through the manual releasing tool. Therefore, the surgical tool makes it possible, for example, to continue the surgical operation or to discontinue the surgical operation to deal even with a case where the power supply is stopped.

### BRIEF DESCRIPTION OF THE DRAWINGS

FIG. 1 is an external diagram of a surgical robot according to a first embodiment.
FIG. 2 is a block diagram of the surgical robot according to the first embodiment.
FIG. 3 is a diagram showing a distal end side of a robot arm and a surgical tool according to the first embodiment.
FIG. 4 is a diagram showing a distal end side of the surgical tool according to the first embodiment.
FIG. 5 is a diagram showing a structure of a main body according to the first embodiment.
FIG. 6 is a diagram showing an appearance of the main body according to the first embodiment.
FIG. 7 is a diagram showing the structure of the main body according to the first embodiment.
FIG. 8 is a diagram showing the structure of the main body according to the first embodiment.
FIG. 9 is a diagram showing an appearance of a main body according to an unclaimed example.

### EXPLANATION OF REFERENCE NUMERALS

10...surgical tool, 11...main body, 12...insertion part, 13...treatment portion, 13A, 13B...movable part, 15 to 17...slider, 18...manual releasing tool, 18A...rack portion, 19...receiver, 19A...rack portion, 20...pinion, 20A...pinion member, 20B ...pinion member

### MODE FOR CARRYING OUT THE INVENTION

For at least a member or portion described with a reference numeral affixed thereto, there is at least one in number unless specified as "one" or the like. In other words, the member or portion may be two or more in number unless specified as "one". The surgical robot and the surgical tool shown in the present disclosure comprises at least components such as members or portions described with reference numerals affixed thereto, and structural portions shown in the drawings.

### (First Embodiment)

### <1. Overview of Surgical Robot>

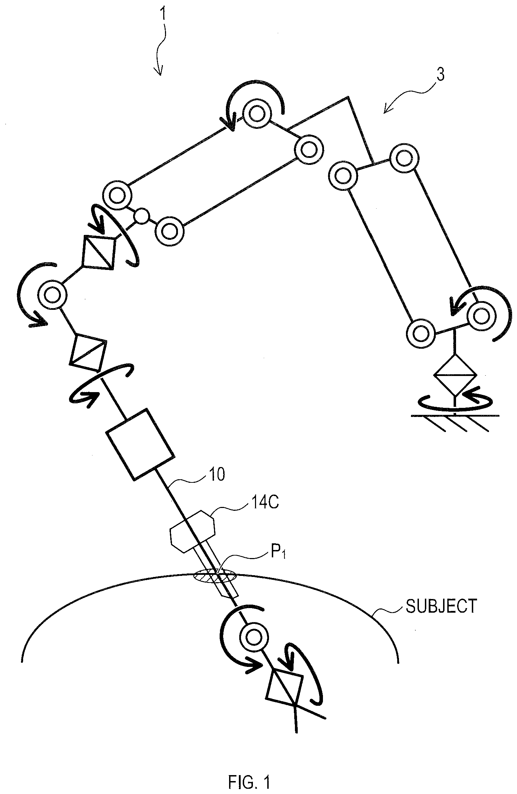

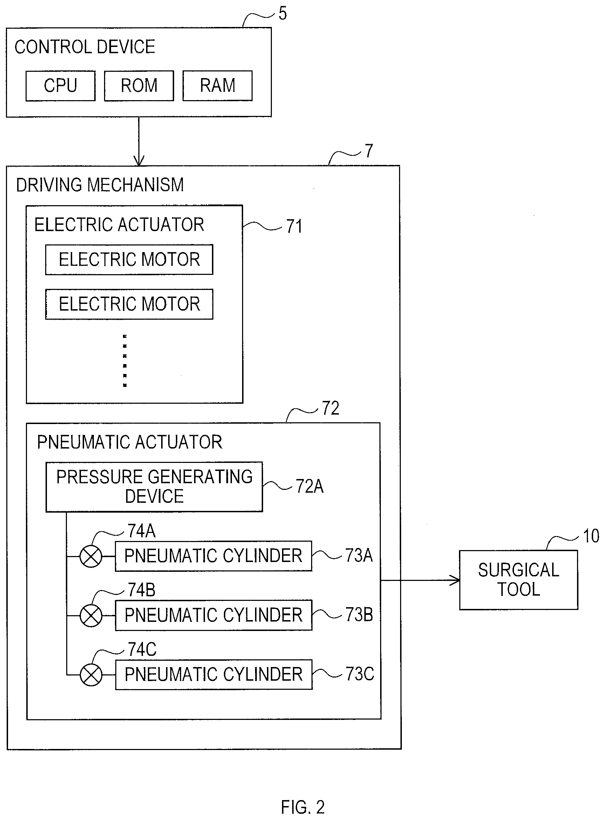

The present embodiment is an example of a surgical robot for use in endoscopic surgical operation. A surgical robot 1 shown in FIG. 1 is an example of a surgical robot for use in endoscopic surgical operation. The surgical robot 1 comprises, as shown in FIG. 2, in addition to a robot arm 3, a control device 5, a driving mechanism 7 and a surgical tool 10.

### <Robot Arm>

As shown in FIG. 1, the robot arm 3 is an example of an arm device that can displace the surgical tool 10 in a state in which the surgical tool 10 is held. Specifically, the robot arm 3 is configured by a link mechanism having two or more joints.

### <Driving Mechanism>

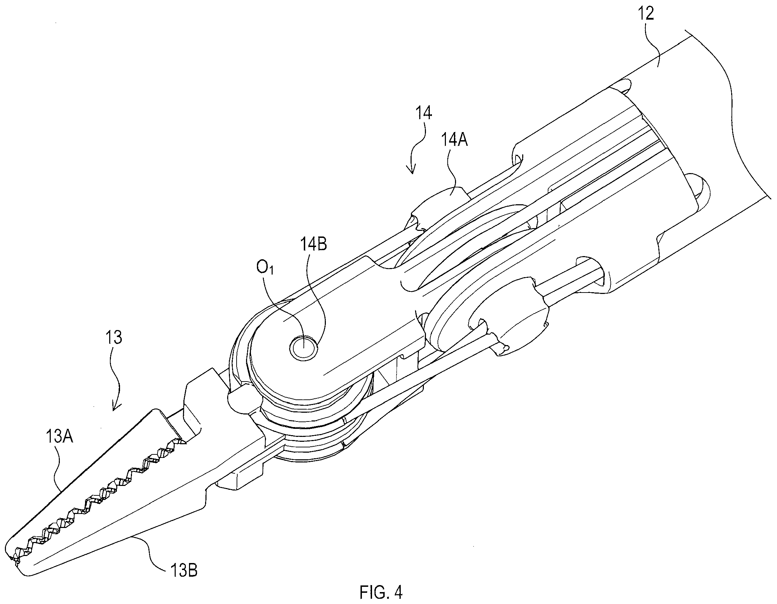

As shown in FIG. 4, the driving mechanism 7 is an example of a driving part for driving movable parts 13A and 13B of the robot arm 3 and the surgical tool 10. The driving mechanism 7 according to the present embodiment comprises an electric actuator 71 and a pneumatic actuator 72, as shown in FIG. 2.

The electric actuator 71 is configured by at least one electric motor and drives each of the joints. In the present embodiment, each joint is provided with an electric motor. Thus, each electric motor is arranged at the robot arm 3.

As shown in FIG. 4, the pneumatic actuator 72 operates at least the movable parts 13A and 13B. As shown in FIG. 2, the pneumatic actuator 72 comprises at least one (three, in the present embodiment) pneumatic cylinders 73A to 73C, a pressure generating device 72A, and the same number of controller solenoid valves 74A to 74C as the pneumatic cylinders.

The pressure generating device 72A is a pressure supply source that supplies compressed air to the pneumatic cylinders 73A to 73C. Each of the pneumatic cylinders 73A to 73C is operated by receiving an air pressure. Each of the controller solenoid valves 74A to 74C controls the air pressure supplied to each of the pneumatic cylinders 73A to 73C, to thereby control operations of the corresponding pneumatic cylinders 73A to 73C.

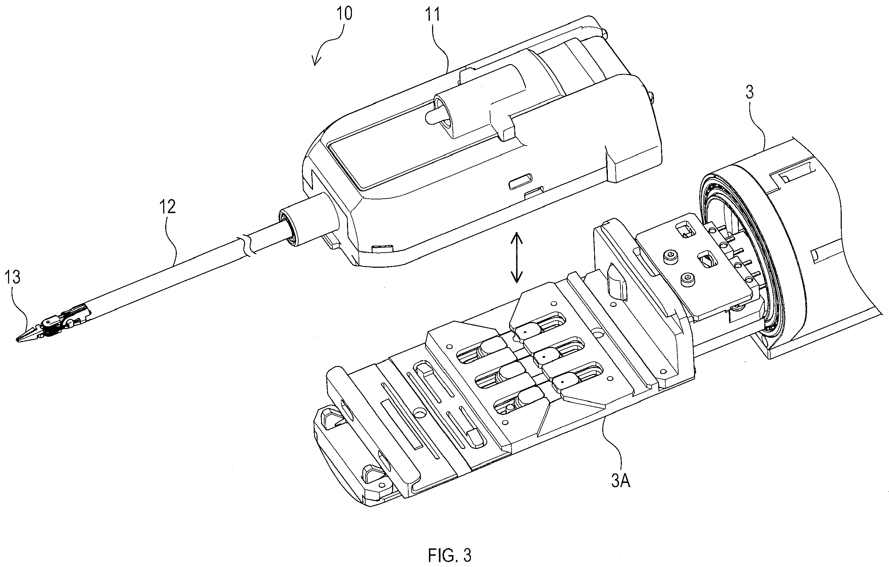

In the present embodiment, at least the pneumatic cylinders 73A to 73C are provided at the robot arm 3. As shown in FIG. 3, the surgical tool 10 is detachably mounted to mounted portion 3A provided at a distal end of the robot arm 3.

### <Control Device>

The control device 5 controls operations of the driving mechanism 7, more specifically the electric actuator 71, the pneumatic actuator 72, and the like. More specifically, the control device 5 controls the controller solenoid valves 74A to 74C. The control device 5 receives a command signal output from a master-side input operation device (not shown) and operates the driving mechanism 7 according to the command signal.

At this time, as shown in FIG. 1, the control device 5 controls the operation of the electric actuator 71 so that a portion of the surgical tool 10 corresponding to an incision position P1 of a subject is immovable. The incision position P1 is a site into which a trocar 14C as shown in FIG. 1 is inserted during endoscopic surgical operation.

The trocar 14C is a cylindrical member. A trocar is also referred to as a trochar. The trocar may be also described a "cannula". An operator inserts the surgical tool 10, such as an endoscope or forceps, into a body of the subject through the trocar 14C, and performs a surgical operation while looking at an image captured by the endoscope.

### <2. Surgical Tool>

### <2.1 Overview of Surgical Tool>

The surgical tool 10 comprises, as shown in FIG. 3, a main body 11, an insertion part 12, and a treatment portion 13. The main body 11 is a part that is detachably mountable to the robot arm 3, more specifically to mounted portion 3A.

The insertion part 12 is a tubular part extending from the main body 11. The insertion part 12 is a part that is inserted into a body through the trocar 14C and formed from a material with a high bending rigidity, such as a metal.

At a distal end portion of the insertion part 12, the treatment portion 13 is provided. As shown in FIG. 4, the treatment portion 13 comprises at least one (in the present embodiment, two) movable parts 13A and 13B.

The movable parts 13A and 13B are parts displaceable with respect to the insertion part 12. Specifically, the surgical tool 10 according to the present embodiment is forceps. In other words, the treatment portion 13 according to the present embodiment is a gripper that can grip a portion to be treated, a suturing needle, or the like.

The two movable parts 13A and 13B can be rotationally displaced or pivoted around a central axis O1 between a "closed position (see, FIG. 4)" and an "open position" in which their distal ends are separated. Thus, the treatment portion 13 can grip a target site or the like.

The treatment portion 13 according to the present embodiment is connected to a distal end of the insertion part 12 via the joint 14. The joint 14 is a mechanism for changing an angle of the treatment portion 13. Specifically, the joint 14 comprises at least one (two, in the present embodiment) rotational shafts 14A and 14B.

The joint 14 can be rotationally displaced around each of the rotational shafts 14A and 14B as a central axis. A first rotational shaft 14A is a shaft perpendicular to a longitudinal direction of the insertion part 12. A second rotational shaft 14B is a shaft perpendicular to the longitudinal direction of the insertion part 12 and the first rotational shaft 14A.

### <2.2 Mechanism for Displacing Movable Part>

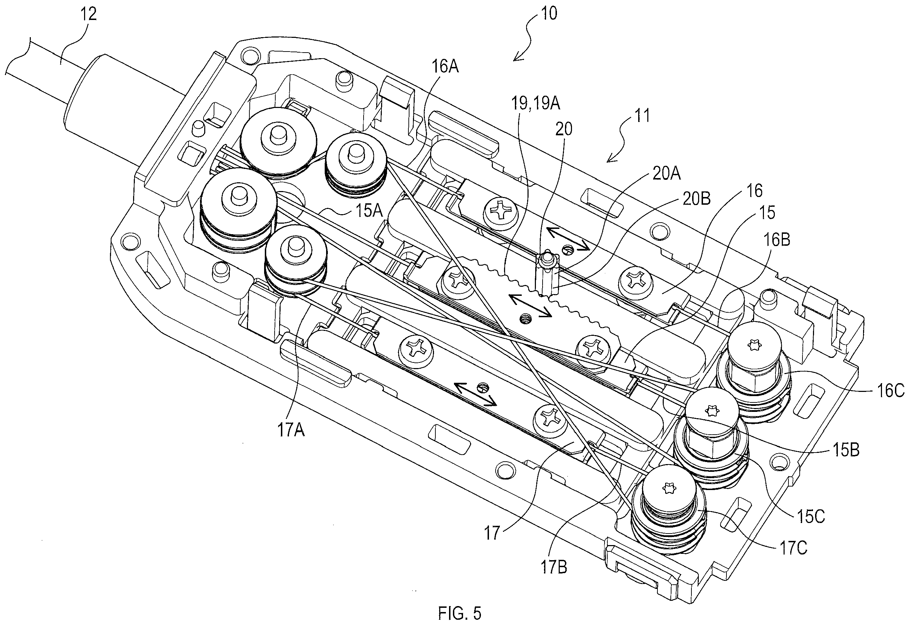

Inside the main body 11, as shown in FIG. 5, at least one (two or more, in the present embodiment) sliders 15 to 17 are accommodated. Each of the sliders 15 to 17 is a member that is reciprocatingly movable along a linear direction.

In the present embodiment, each of the sliders 15 to 17 is displaced along a direction parallel to the longitudinal direction (specifically, an arrow direction shown in FIG. 5) of the insertion part 12. Each of the sliders 15 to 17 is displaced by receiving a driving force from the pneumatic cylinders 73A to 73C as shown in FIG. 2.

The slider 15 makes a reciprocating motion, thereby generating a driving force to displace the movable part 13A. A first end 15A of a driving wire 15W and a second end 15B of the driving wire 15W are respectively connected to a first end and a second end of the slider 15 along a displacement direction.

The slider 16 makes a reciprocating motion, thereby generating a driving force to displace the movable part 13B. A first end 16A of a driving wire 16W and a second end 16B of the driving wire 16W are respectively connected to a first end and a second end of the slider 16 along a displacement direction.

The driving wire 15W is directly or indirectly connected to the movable part 13A. Therefore, when the slider 15 is displaced, the movable part 13A is displaced in conjunction with the displacement. Specifically, when the slider 15 makes the reciprocating motion, the movable part 13A of the treatment portion 13 configuring the gripper is displaced around the second rotational shaft 14B in conjunction with the reciprocating motion.

The driving wire 16W is directly or indirectly connected to the movable part 13B. Therefore, when the slider 16 is displaced, the movable part 13B is displaced in conjunction with the displacement. Specifically, when the slider 16 makes the reciprocating motion, the movable part 13B of the treatment portion 13 configuring the gripper is displaced around the second rotational shaft 14B in conjunction with the reciprocating motion.

In a case in which turning directions of the movable part 13A and the movable part 13B are opposite to each other, the treatment portion 13 opens and closes. In a case in which the turning directions of the movable part 13A and the movable part 13B are the same, the treatment portion 13 is displaced around the second rotational shaft 14B.

The slider 17 generates a driving force to operate the joint 14. A first end 17A of a driving wire 17W and a second end 17B of the driving wire 17W are respectively connected to a first end and a second end of the slider 17 along a displacement direction.

The driving wire 17W displaces the treatment portion 13 and the second rotational shaft 14B around the first rotational shaft 14A. Thus, the joint 14 is operated in conjunction with displacement of the sliders 15, 16 and 17.

A pulley 15C is a fixed pulley that redirects the driving wire 15W. A pulley 16C is a fixed pulley that redirects the driving wire 16W. A pulley 17C is a fixed pulley that redirects the driving wire 17W.

### <2.3 Manual Release of Treatment Portion (Gripper)>

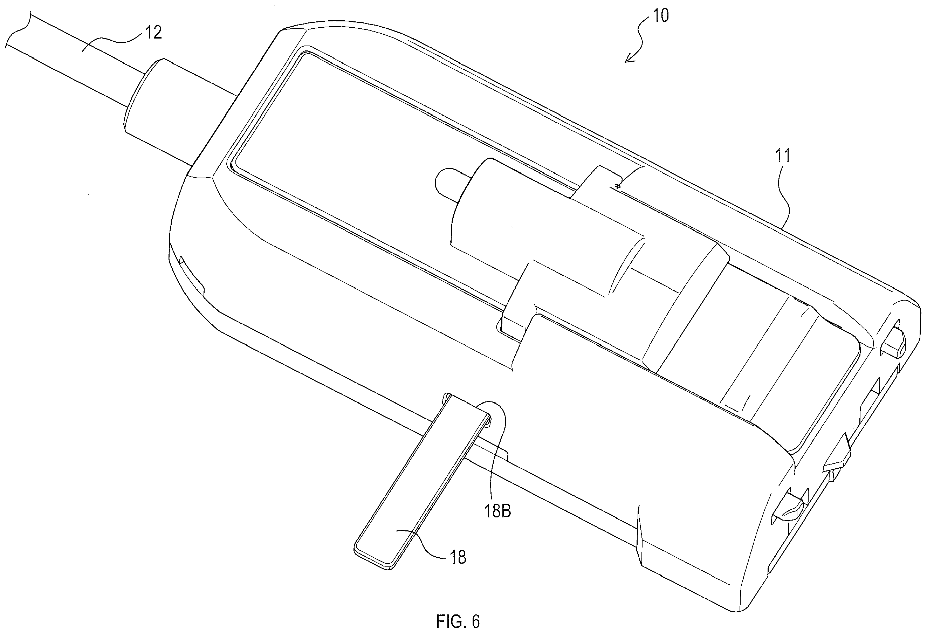

As shown in FIG. 6, the surgical tool 10 comprises a manual releasing tool 18. The manual releasing tool 18 is a member for displacing the movable part 13A and is detachable with respect to the main body 11.

FIG. 6 shows a state in which the manual releasing tool 18 is mounted to the main body 11. FIG. 3 shows a state in which the manual releasing tool 18 is not mounted. In the state in which the manual releasing tool 18 is not mounted, the manual releasing tool 18 is held by, for example, the robot arm 3.

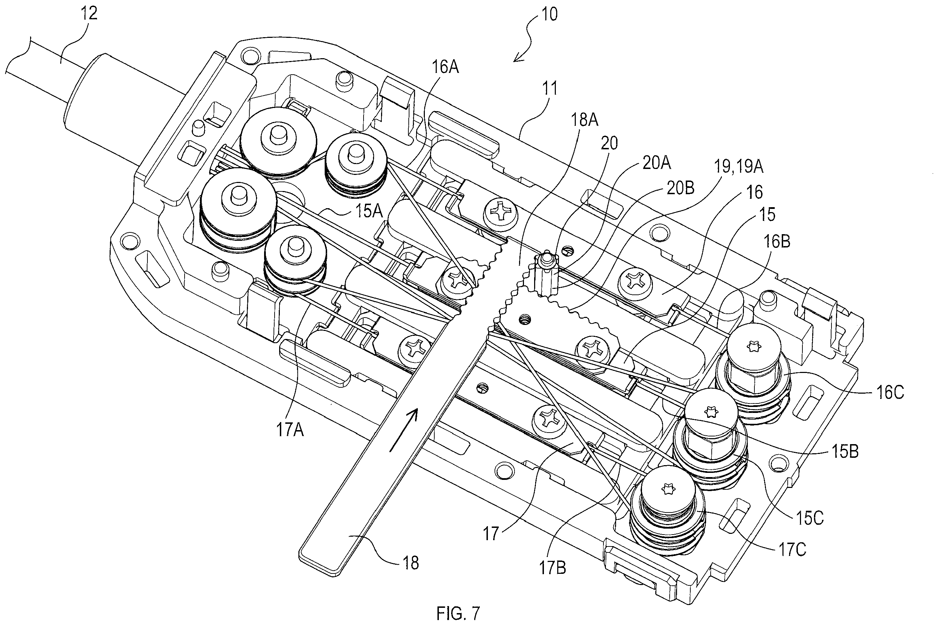

On the slider 15, as shown in FIG. 5, a receiver 19 is provided. The receiver 19 is a part that directly or indirectly receives a driving force from the manual releasing tool 18. In the present embodiment, the receiver 19 indirectly receives the driving force from the manual releasing tool 18, as shown in FIG. 7.

The receiver 19 according to the present embodiment is configured by a rack portion 19A that extends along a direction parallel to a sliding direction of the slider 15. Here, a rack is a sector gear whose curvature radius is infinite. Hereinafter, the receiver 19 will be also referred to as the rack portion 19A. In other words, the receiver 19 comprises the rack portion 19A.

The manual releasing tool 18 according to the present embodiment is mounted to the main body 11 so as to be displaceable along a direction intersecting (in the present embodiment, for example, perpendicular to) the displacement direction of the slider 15. The manual releasing tool 18 is provided with a rack portion 18A on its distal end side in a direction of insertion.

The main body 11 is provided with a pinion member 20A that is engageable with the rack portion 18A and a pinion member 20B that is engageable with the rack portion 19A. The pinion member 20A and the pinion member 20B are integrated together in such a manner that their respective rotational axes coincide. Hereinafter, the two integrated pinion members 20A and 20B will be referred to as a pinion 20. In other words, the pinion 20 comprises the pinion member 20A and the pinion member 20B.

Thus, when the manual releasing tool 18 is displaced in a state in which the manual releasing tool 18 and the pinion member 20A are engaged with each other, the pinion member 20B is rotated, whereby the rotation is transmitted to the rack portion 19A.

In the pinion 20, a position of the rotational axis is immovable relative to the main body 11. Thus, when the manual releasing tool 18 is displaced in the state in which the manual releasing tool 18 and the pinion 20 are engaged with each other, the slider 15 is displaced along a linear direction by receiving a driving force from the rack portion 19A.

The pinion 20 functions as a transmitter that converts a mounting force received by the manual releasing tool 18 when the manual releasing tool 18 is mounted to the main body 11, and transmits the converted mounting force to the receiver 19. In the present embodiment, when the manual releasing tool 18 is displaced in the direction of insertion (in other words, a direction of an arrow in FIG. 7), the movable part 13A is displaced so as to open the treatment portion 13.

The mounting force corresponds to one example of an operating force.

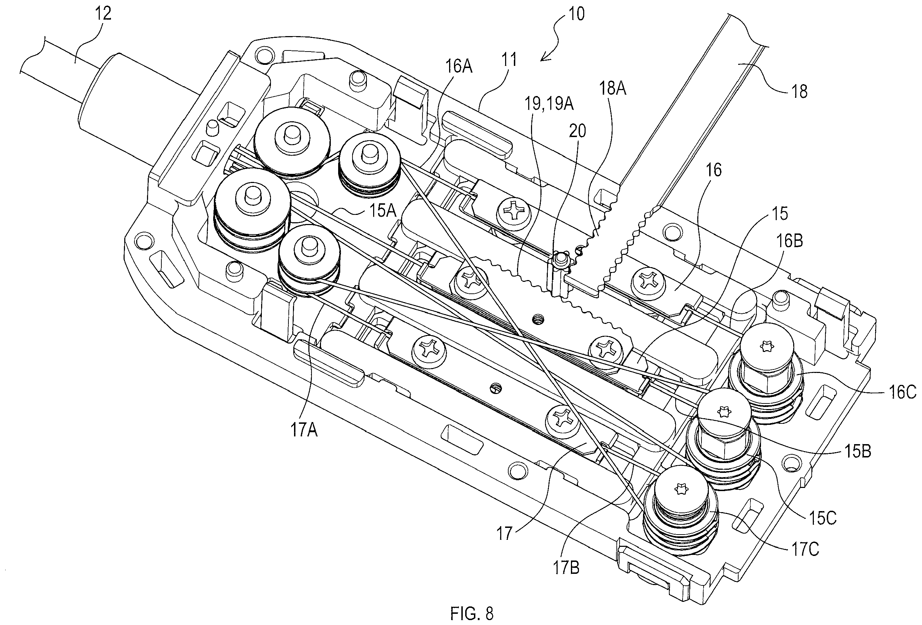

As shown in FIG. 8, the surgical tool 10 according to the present embodiment allows the user to insert the manual releasing tool 18 into the main body 11 from a position different from a position shown in FIG. 7. The main body 11 according to the present embodiment is provided with two or more mounted portions (mounted portions configured such that the manual releasing tool 18 can be mounted to it) 18B as shown in FIG. 6 (however, only one mounted portion 18B is shown in FIG. 6).

In two or more (two, in the present embodiment) mounted portions 18B, a first mounted portion 18B is arranged on a first side of the main body 11, and a second mounted portion 18B is arranged on a second side of the main body 11. The second mounted portion 18B is arranged at a position rotated by 180 degrees with respect to the first mounted portion 18B about the rotational axis of the pinion 20.

### <3. Features of Surgical Robot (Particularly, Surgical Tool) According to the Present Embodiment>

The surgical tool 10 according to the present embodiment comprises the manual releasing tool 18 for displacing the movable part 13A, the manual releasing tool 18 being detachably mountable to the main body 11.

Thus, in the surgical tool 10, when the user mounts the manual releasing tool 18 to the main body 11, the user can displace the movable part 13A by manual operation through the manual releasing tool 18. Therefore, the surgical tool makes it possible, for example, to continue the surgical operation or to discontinue the surgical operation to deal even with a case where the power supply is stopped.

The slider 15 is provided with the receiver 19 that directly or indirectly receives the driving force from the manual releasing tool 18. The slider 15 can be displaced along the linear direction by receiving the driving force from the receiver 19. Thus, the user can reliably displace the movable part 13A by manual operation through the manual releasing tool 18.

In the present embodiment, when the manual releasing tool 18 is mounted to the main body 11, the rack portion 18A and the pinion member 20A are engaged with each other, whereby the slider 15 is displaced. Specifically, in the present embodiment, the mounting force received by the manual releasing tool 18 is converted into a force to displace the slider 15 at the transmitter and the receiver 19. The pinion 20 corresponds to one example of the transmitter.

Therefore, the user can displace the movable part 13A by only mounting the manual releasing tool 18 to the main body 11 in an inserting manner. This makes it possible for the user to quickly and reliably open the treatment portion 13. The treatment portion 13 corresponds to one example of the gripper.

### (Second Embodiment)

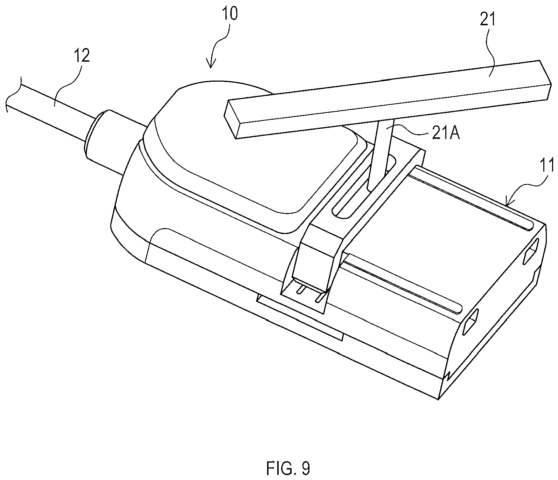

The manual releasing tool 18 according to the above-described embodiment is configured to comprise the rack portion 18A that is engaged with the pinion member 20A. On the other hand, in an unclaimed example, as shown in FIG. 9, a manual releasing tool 21 according to the present embodiment is a member connectable with the pinion member 20B. For this reason, in the present embodiment, the rack portion 18A and the pinion member 20A are not provided. The pinion member 20B is included in the pinion 20.

The pinion member 20B is provided with a connecting member (not shown) that is detachably connectable with an axial member 21A shown in FIG. 9 for the manual releasing tool 21. The user can displace the movable part 13A by rotating the manual releasing tool 21 in a state in which the axial member 21A of the manual releasing tool 21 is connected to the connecting member.

In yet another unclaimed example, the transmitter may be omitted, and the pinion member 20B may be configured to be arranged in the axial member 21A of the manual releasing tool 21. According to this configuration, when the manual releasing tool 21 is mounted to the main body 11, and the pinion member 20B of the axial member 21A is engaged with the rack portion 19A, the slider 15 goes into a state in which it is displaceable by manual operation.

### (Other Embodiments)

The sliders 15 to 17 according to the above-described embodiment each is configured to make the reciprocating motion along a direction parallel to the longitudinal direction of the insertion part 12. However, the present disclosure is not limited to this. For example, the sliders 15 to 17 each may be configured to make the reciprocating motion along a direction intersecting the longitudinal direction of the insertion part 12.

In the above-described embodiment, the pinion member 20B and the rack portion 19A are provided, and the pinion member 20B and the rack portion 19A are configured to be engaged with each other, whereby operating forces of the manual releasing tools 18 and 21 are transmitted to the slider 15.

In an unclaimed example, the present disclosure may be configured such that the pinion member 20B and the rack portion 19A are be omitted, and a friction force generated at a contact area between the transmitter and the slider 15 is used to transmit the operating forces to the slider 15.

In the above-described embodiment, the operating forces of the manual releasing tools 18 and 21 are configured to be transmitted to the slider 15 through the transmitter. In an unclaimed example, the manual releasing tools 18 and 21 may be configured to be detachably connectable to the slider 15.

In this case, the slider 15 may be configured to be displaced by the user displacing the manual releasing tools 18 and 21 along the sliding direction in a state in which the manual releasing tools 18 and 21 are connected to the slider 15.

In the above-described embodiment, the movable part 13A is configured to be displaced by displacing the slider 15 with the manual releasing tools 18 and 21. In an unclaimed example the present disclosure may be configured such that a member other than the slider 15 such as the driving wire 15W can be operated with the manual releasing tools 18 and 21.

In the above-described embodiment, mounting directions of the manual releasing tools 18 and 21 are perpendicular to the sliding direction of the slider 15. However, the present disclosure is not limited to this. For example, the mounting directions of the manual releasing tools 18 and 21may be parallel to the sliding direction.

In the above-described embodiment, the two mounted portions 18B to which the manual releasing tool 18 can be mounted are provided. However, the present disclosure is not limited to this. For example, the present disclosure may be configured such that one, or three or more mounted portions 18B are provided.

In the above-described embodiment, the displacement of the slider 15 is configured to be transmitted to the movable part 13A through the driving wire 15W. However, the present disclosure is not limited to this. For example, the displacement of the slider 15 may be configured to be transmitted to the movable part 13A through a push-pull wire or a geared cable.

The treatment portion 13 according to the above-described embodiment is configured by a so-called gripper and is a portion separate from the joint 14. However, the present disclosure is not limited to this. For example, the present disclosure may be configured such that the treatment portion 13 is configured by a component other than the gripper or may be configured such that the treatment portion 13 and the joint 14 are integrated together; in other words, the present disclosure may be configured such that the joint 14 can also be operated with the manual releasing tools 18 and 21.

In the above-described embodiment, the manual releasing tools 18 and 21 are configured to displace the slider 15. However, the present disclosure is not limited to this. For example, the present disclosure may be configured such that the manual releasing tools 18 and 21 can displace the sliders 16 and 17, as well.

In the aforementioned embodiment, the slider 15 is configured to be operated through the manual releasing tools 18 and 21. In an unclaimed example, the slider 15 may be provided with a projecting operating part and may be configured such that a distal end side of the operating part protrudes from the main body 11 enough that it can be operated by the user. In this configuration, the manual releasing tools 18 and 21 are not necessary.

Further, the present disclosure is only required to be consistent with the scope of the independent claim, and thus is not limited to the aforementioned embodiments. Accordingly, the present disclosure may be configured in combination of at least two of the aforementioned embodiments, or may be configured without some of the components illustrated in the drawings or described with reference numerals in the aforementioned embodiments.

## Claims

1. A surgical tool (10) mounted to a surgical robot (1), the surgical tool (10) comprising:
a treatment portion (13) used in surgical treatment, the treatment portion (13) including a movable part (13A, 13B) that can be displaced;
a slider (15, 16, 17) that generates a driving force to displace the movable part (13A, 13B), the slider (15, 16, 17) generating the driving force through reciprocating motion along a linear direction;
a manual releasing tool (18) for displacing the movable part (13A, 13B), the manual releasing tool (18) being detachably mountable to a main body (11) of the surgical tool (10) at which the slider (15, 16, 17) is accommodated; **characterised in that** the surgical tool comprises
a transmitter (20) arranged in the main body (11), the transmitter (20) converting a mounting force received from the manual releasing tool (18), when the manual releasing tool (18) is mounted to the main body (11), and transmitting the converted mounting force to a receiver (19),
wherein the transmitter (20) includes a pinion member (20A) that is engageable with a rack portion (18A) provided in the manual releasing tool (18), and a second pinion member (20B) that is engageable with a rack portion (19A) configuring the receiver,
wherein the slider (15, 16, 17) is provided with the receiver (19) that directly or indirectly receives the driving force from the manual releasing tool (18), and
wherein the slider (15, 16, 17) can be displaced along a linear direction by receiving the driving force from the receiver (19).

2. The surgical tool (10) according to claim 1,
wherein the manual releasing tool (18) is mountable to the main body (11) so as to be displaceable along a direction intersecting a displacement direction of the slider (15, 16, 17).

3. The surgical tool (10) according to any one of claims 1 to 2, the main body (11) is provided with two or more mounted portions (18B) to which the manual releasing tool (18) can be mounted.

## Patentansprüche

1. Chirurgisches Werkzeug (10), das an einem chirurgischen Roboter (1) montiert ist, wobei das chirurgische Werkzeug (10) enthält:
einen Behandlungsabschnitt (13), der bei einer chirurgischen Behandlung verwendet wird, wobei der Behandlungsabschnitt (13) einen beweglichen Teil (13A, 13B) einschließt, der versetzt werden kann;
einen Schieber (15, 16, 17), der eine Antriebskraft erzeugt, um den beweglichen Teil (13A, 13B) zu verschieben, wobei der Schieber (15, 16, 17) die Antriebskraft durch Hin- und Herbewegung entlang einer linearen Richtung erzeugt;
ein manuelles Freigabewerkzeug (18) zum Verschieben des beweglichen Teils (13A, 13B), wobei das manuelle Freigabewerkzeug (18) lösbar an einem Hauptkörper (11) des chirurgischen Werkzeugs (10) montierbar ist, an dem der Schieber (15, 16, 17) untergebracht ist; **dadurch gekennzeichnet, dass** das chirurgische Werkzeug enthält
einen Überträger (20), der in dem Hauptkörper (11) angeordnet ist, wobei der Überträger (20) eine von dem manuellen Freigabewerkzeug (18) aufgenommene Montagekraft umwandelt, wenn das manuelle Freigabewerkzeug (18) an dem Hauptkörper (11) montiert ist, und die umgewandelte Montagekraft an eine Aufnahmeeinrichtung (19) überträgt,
wobei der Überträger (20) ein Zahnradelement (20A), das mit einem Zahnstangenabschnitt (18A) in Eingriff bringbar ist, der in dem manuellen Freigabewerkzeug (18) bereitgestellt ist, und ein zweites Zahnradelement (20B) einschließt, das mit einem Zahnstangenabschnitt (19A) in Eingriff bringbar ist, der die Aufnahmeeinrichtung konfiguriert,
wobei der Schieber (15, 16, 17) mit der Aufnahmeeinrichtung (19) bereitgestellt ist, die direkt oder indirekt die Antriebskraft von dem manuellen Freigabewerkzeug (18) aufnimmt, und
wobei der Schieber (15, 16, 17) entlang einer linearen Richtung durch Aufnehmen der Antriebskraft von der Aufnahmeeinrichtung (19) verschoben werden kann.

2. Chirurgisches Werkzeug (10) nach Anspruch 1,
wobei das manuelle Freigabewerkzeug (18) an dem Hauptkörper (11) montierbar ist, um entlang einer Richtung verschiebbar zu sein, die eine Verschieberichtung des Schiebers (15, 16, 17) schneidet.

3. Chirurgisches Werkzeug (10) nach einem der Ansprüche 1 bis 2, wobei der Hauptkörper (11) mit zwei oder mehr angebrachten Abschnitten (18B) bereitgestellt ist, an denen das manuelle Freigabewerkzeug (18) montiert werden kann.

## Revendications

1. Un outil chirurgical (10) monté sur un robot chirurgical (1), l'outil chirurgical (10) comprenant :
une partie de traitement (13) utilisée dans le traitement chirurgical, la partie de traitement (13) comprenant une partie mobile (13A, 13B) qui peut être déplacée ;
un curseur (15, 16, 17) qui génère une force motrice pour déplacer la partie mobile (13A, 13B), le curseur (15, 16, 17) générant la force motrice par un mouvement alternatif le long d'une direction linéaire ;
un outil de déverrouillage manuel (18) pour déplacer la partie mobile (13A, 13B), l'outil de déverrouillage manuel (18) pouvant être monté de manière amovible sur un corps principal (11) de l'outil chirurgical (10), sur lequel le curseur (15, 16, 17) est logé ; **caractérisé en ce que** l'outil chirurgical comprend
un transmetteur (20) disposé dans le corps principal (11), le transmetteur (20) convertissant une force de montage reçue de l'outil de déverrouillage manuel (18), lorsque l'outil de déverrouillage manuel (18) est monté sur le corps principal (11), et transmettant la force de montage convertie à un récepteur (19),
dans lequel le transmetteur (20) comprend un élément de pignon (20A) qui peut être engagé avec une partie de crémaillère (18A) fournie dans l'outil de déverrouillage manuel (18), et un deuxième élément de pignon (20B) qui peut être engagé avec une partie de crémaillère (19A) configurant le récepteur,
dans lequel le curseur (15, 16, 17) est muni du récepteur (19) qui reçoit directement ou indirectement la force motrice de l'outil de déverrouillage manuel (18), et
dans lequel le curseur (15, 16, 17) peut être déplacé le long d'une direction linéaire en recevant la force motrice du récepteur (19).

2. L'outil chirurgical (10) selon la revendication 1,
dans lequel l'outil de déverrouillage manuel (18) peut être monté sur le corps principal (11) de manière à pouvoir être déplacé le long d'une direction coupant une direction de déplacement du curseur (15, 16, 17).

3. L'outil chirurgical (10) selon l'une quelconque des revendications 1 à 2, dans lequel le corps principal (11) est muni d'au moins deux parties montées (18B) sur lesquelles l'outil de déverrouillage manuel (18) peut être monté.
